(19) **Europäisches Patentamt / European Patent Office / Office européen des brevets**

(11) **EP 2 692 275 A1**

(12) **EUROPEAN PATENT APPLICATION**
published in accordance with Art. 153(4) EPC

(43) Date of publication:
05.02.2014 Bulletin 2014/06

(21) Application number: **12764519.0**

(22) Date of filing: **10.02.2012**

(51) Int Cl.:
*A61B 1/00* (2006.01)     *A61B 1/04* (2006.01)
*A61B 5/1455* (2006.01)

(86) International application number:
**PCT/JP2012/053093**

(87) International publication number:
**WO 2012/132571 (04.10.2012 Gazette 2012/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.03.2011 JP 2011072216**

(71) Applicants:
- **HOYA Corporation**
  **Tokyo 161-8525 (JP)**
- **Kyushu University**
  **National University Corporation**
  **Higashi-ku**
  **Fukuoka-shi**
  **Fukuoka 812-8581 (JP)**

(72) Inventors:
- **CHIBA, Toru**
  **Tokyo 161-8525 (JP)**
- **HASHIZUME, Makoto**
  **Fukuoka-shi, Fukuoka 812-8581 (JP)**
- **MATSUMOTO, Takayuki**
  **Fukuoka-shi, Fukuoka 812-8581 (JP)**
- **KONISHI, Kozo**
  **Fukuoka-shi, Fukuoka 812-8581 (JP)**
- **TOMIKAWA, Morimasa**
  **Fukuoka-shi, Fukuoka 812-8581 (JP)**
- **MURATA, Masaharu**
  **Fukuoka-shi, Fukuoka 812-8581 (JP)**
- **AKAHOSHI, Tomohiko**
  **Fukuoka-shi, Fukuoka 812-8581 (JP)**

(74) Representative: **Schaumburg, Thoenes, Thurn, Landskron, Eckert**
**Postfach 86 07 48**
**81634 München (DE)**

(54) **DIAGNOSTIC SYSTEM**

(57) A diagnostic system comprises: a spectral imaging means that captures a spectral image within a predetermined wavelength range in a body cavity and obtains spectral image data; an image processing means that receives the spectral image data, determines an index value indicating a region that is highly likely to be a lesion from the spectral image data, and generates and outputs an extracted lesion image based on the index value; and a monitor on which the extracted lesion image is displayed. The image processing means performs multiple regression analysis for each pixel of the spectral image with the spectral image data as a dependent variable and respective light absorption properties of oxyhemoglobin and deoxyhemoglobin as independent variables, and determines the index value based on respective concentrations of the oxyhemoglobin and the deoxyhemoglobin.

FIG. 7

**Description**

**TECHNICAL FIELD**

The present invention relates to a diagnostic system configured to display an image of a region that is highly likely to be a lesion in a living tissue.

**BACKGROUND ART**

Recently, an electronic endoscope having a function as a spectrometer has been proposed as described, for example, in Japanese Patent Provisional Publication No. JP2004-321792A. By using such an electronic endoscope, it is possible to obtain the spectral property (the distribution of light absorption property for each frequency) of a living tissue such as a mucous membrane of a digestive organ, e.g., a stomach or a rectum. It is known that the spectral property of a substance reflects information concerning the types or densities of components contained in the vicinity of the surface layer of a living tissue being an observation target, which is established in a field belonging to academic frameworks of analytical chemistry. It is also known in this field that the spectral property of a substance consisting of a composite is information obtained by superimposing the spectral properties of essential components constituting the composite.

A lesion living tissue may contain a substance having a chemical configuration that is rarely contained in a healthy living tissue. Therefore, a spectral property of a living tissue containing a lesion is different from a spectral property of a living tissue containing only a healthy region. Since the spectral properties of the healthy region and the lesion are different from each other as described above, it becomes possible to judge whether or not a living tissue contains a lesion by comparing the spectral property of the healthy region with that of the lesion.

**SUMMARY OF THE INVENTION**

As described above, researches have been carried out to determine the presence of lesions in a living tissue using the differences of in vivo spectral properties obtained from living bodies. However, the known researches have not proposed any practical diagnostic methods to generate images for determining where in the living tissue there is a change in the spectral property caused by lesions, and identify the position and the extent of a lesion while comparing the lesion with the surrounding tissues.

The present invention is made to solve the above described problem. That is, the object of the present invention is to provide a diagnostic system configured to display an image of a region that is highly likely to be a lesion.

To achieve the above described object, the diagnostic system according to the invention includes a spectral imaging means which captures a spectral image within a prescribed wavelength range in a body cavity and obtains spectral image data, an image processing means that obtains the spectral image data, determines an index value indicating a region that is highly likely to be a lesion from the spectral image data, and generates and outputs an extracted lesion image based on the index value, and a monitor on which the extracted lesion image is displayed. The image processing means performs multiple regression analysis for each pixel of the spectral image with the spectral image as a dependent variable and respective light absorption properties of oxyhemoglobin and deoxyhemoglobin as independent variables, and determines the index value on the basis of respective concentrations of the oxyhemoglobin and the deoxyhemoglobin.

As a result of the multiple regression analysis with the spectral image data as a dependent variable and the light absorption properties of the oxyhemoglobin and the deoxyhemoglobin as independent variables, the inventors of the present invention found out that the spectral image data can be explained using the light absorption property of the oxyhemoglobin, the light absorption property of the deoxyhemoglobin and an influence of light scattering, and that the concentration of the oxyhemoglobin at a lesion is higher than that at a healthy region. The present invention uses the aforementioned properties, and is configured to perform multiple regression analysis for each pixel of the spectral image with the spectral image data as a dependent variable and the light absorption properties of the oxyhemoglobin and the deoxyhemoglobin as independent variables, to determine the index value on the basis of the concentrations of the oxyhemoglobin and the deoxyhemoglobin, and to output the extracted lesion images on the basis of the determined index value on the monitor. Thus, according to the aforementioned configuration, it is possible to assist detection and diagnostic of lesions by displaying on the monitor a region where the oxyhemoglobin concentration differs from those of the surrounding areas as the extracted lesion image.

The image processing means may be configured to determine, as the index value, a ratio between the concentration of the oxyhemoglobin and the concentration of the deoxyhemoglobin. With this configuration, it becomes possible to precisely judge which region is highly likely to be a lesion.

The image processing means may be configured to assign to each pixel of the spectral image a predetermined color according to the index value to generate the extracted lesion image. The image processing means may also include a comparing means that compares the index value with a predetermined threshold value, and a binary image generating means that generates a binary image based on a result of the comparison by the comparing means. The extracted lesion image may also be generated from the binary image. With this configuration, it becomes possible to easily discriminate the lesion from the healthy region.

The image processing means may be configured to generate a color image by synthesizing data, of the spectral image data, having wavelength bands for blue color, green color and red color, and output the color image. Further, on the monitor, the color image and the extracted lesion image may be displayed side by side. With this configuration, it becomes possible to easily determine which region is highly likely to be a lesion by comparison between the color image and the extracted lesion image of a living tissue of which the spectral image is captured by the spectral imaging means.

The image processing means may be configured to determine the index value from data, of the spectral image data, of a wavelength band of 500 nm to 590 nm that is an absorption wavelength band of the oxyhemoglobin and the deoxyhemoglobin. With this configuration, it becomes possible to calculate multiple regression coefficients faster and more accurately.

Preferably, the predetermined wavelength range may be from 400 nm to 800 nm, and the spectral image may include a plurality of images captured at every predetermined wavelength range of 1 nm to 10 nm.

As described above, according to the invention, since an image of a region that is highly likely to be a lesion is displayed, it is possible to shorten a time for the diagnostic, and easily confirm and identify regions that need to be operated.

**BRIEF DESCRIPTION OF THE DRAWINGS**

[Fig. 1] Fig. 1 is a block diagram illustrating a diagnostic system 1 according to an embodiment of the invention.
[Fig. 2] Fig. 2 is a graph illustrating spectral image data of gastric mucosa obtained from the diagnostic system 1 according to the embodiment of the invention. Fig. 2A illustrates a spectrum of a pixel corresponding to a lesion of the gastric mucosa, and Fig. 2B illustrates a spectrum of a pixel corresponding to a healthy region thereof.
[Fig. 3] Fig. 3 is a graph illustrating absorption properties of hemoglobin.
[Fig. 4] Fig. 4 is a graph illustrating a result of multiple regression analysis on the spectral image data of the gastric mucosa shown in Fig.2. Fig. 4A illustrates a result of multiple regression analysis on a spectrum of a pixel corresponding to the lesion of the gastric mucosa shown in Fig. 2A, and Fig. 4B illustrates a result of multiple regression analysis on a spectrum of a pixel corresponding to the healthy region thereof shown in Fig. 2B.
[Fig. 5] Fig. 5 is a graph illustrating an example of multiple regression coefficients P1 and P2 obtained from multiple regression analysis on the spectral image data of the gastric mucosa shown in Fig. 2.
[Fig. 6] Fig. 6 is a graph illustrating an example of multiple regression coefficients P1 and P2 obtained from multiple regression analysis on the spectral image data of the gastric mucosa shown in Fig. 2.
[Fig. 7] Fig. 7 is a flowchart illustrating an image generating process performed by an image processing unit 500 in the embodiment of the invention.
[Fig. 8] Fig. 8 is a diagram illustrating a color image and an extracted lesion image displayed on an image display device 300 by the image generating process shown in Fig. 7.

**EMBODIMENTS FOR CARRYING OUT THE INVENTION**

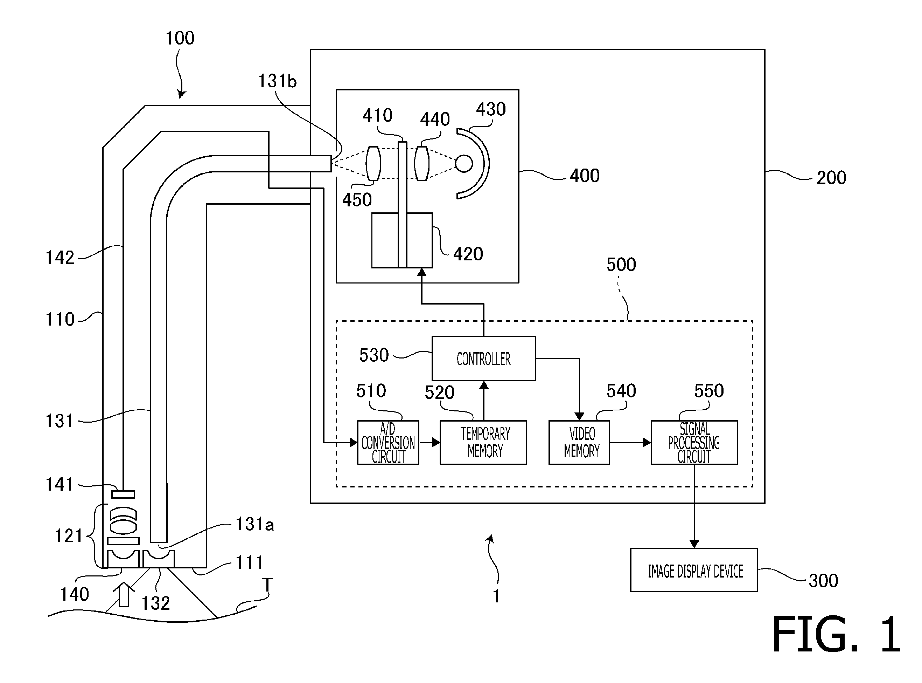

In the following, an embodiment according to the invention is described with reference to the accompanying drawings. Fig. 1 is a block diagram of a diagnostic system 1 according to the embodiment of the invention. The diagnostic system 1 according to the embodiment generates indicative images which are referred to by doctors when diagnosing diseases of digestive organs such as a stomach or a rectum. The diagnostic system 1 has an electronic endoscope 100, a processor 200 for the electronic endoscope and an image display device 300. In the processor 200 for the electronic endoscope, a light source unit 400 and an image processing unit 500 are accommodated.

The electronic endoscope 100 has an insertion tube 110 to be inserted into a body cavity, and an objective optical system 121 is provided at a tip portion (an insertion tube tip portion) 111 of the insertion tube 110. An image of a living tissue T around the insertion tube tip portion 111 is formed by the objective optical system 121 on a light-receiving surface of an image pick-up device 141 accommodated in the insertion tube tip portion 111.

The image pickup device 141 periodically (e.g., at intervals of 1/30 seconds) outputs image signals corresponding to the images formed on the light-receiving surface. The image signals outputted by the image pickup device 141 are transmitted to the image processing unit 500 of the processor 200 for the electronic endoscope via a cable 142.

The image processing unit 500 has an A/D conversion circuit 510, a temporary memory 520, a controller 530, a video memory 540 and a signal processing circuit 550. The A/D conversion circuit 510 executes A/D conversion for the image signals transmitted from the image pickup device 141 of the electronic endoscope 100 via the cable 142 to output digital image data. The digital image data outputted from the A/D conversion circuit 510 is transmitted to and stored in the temporary memory 520. The controller 530 processes a piece of or a plurality of pieces of image data stored in the temporary memory 520 to generate one piece of display image data, and transmits the display image data to the video memory 540. For example, the controller 530 generates display image data such as display image data generated from a piece of image data, display image data in which a plurality of pieces of image data are arranged and displayed, display image data in which an image is obtained by subjecting a plurality of pieces of image data to image processing, or display image data in which a graph obtained as a result of the image processing is displayed, and stores them in the video memory 540. The signal processing circuit 550 converts the display image data stored in the video memory 540 into video signals having a predetermined format (e.g., NTSC format), and outputs the video signals. The video signals outputted from the signal processing circuit 550 are inputted to the image display device 300. As a result, endoscopic images captured by the electronic endoscope 100 are displayed on the image display device 300.

A light guide 131 is provided in the electronic endoscope 100. A tip portion 131a of the light guide 131 is arranged close to the insertion tube tip portion 111, and a proximal end portion 131b of the light guide 131 is connected to the processor 200 for the electronic endoscope. The processor 200 for the electronic endoscope includes therein the light source unit 400 (described later) having a light source 430 generating a large amount of white light, e.g., a Xenon lamp. The light generated by the light source unit 400 is incident on the proximal end portion 131b of the light guide 131. The light which is incident on the proximal end portion 131b of the light guide 131 is guided to the tip portion 131a through the light guide 131, and is emitted from the tip portion 131a. A lens 132 is provided in the vicinity of the tip portion 131a of the light guide 131 in the insertion tube tip portion 111 of the electronic endoscope 100. The light emitted from the tip portion 131a of the light guide 131 passes through the lens 132, and illuminates the living tissue T near the insertion tube tip portion 111.

As described above, the processor 200 for the electronic endoscope has both a function as a video processor processing the image signals outputted from the image pickup device 141 of the electronic endoscope 100, and a function as a light source device supplying illumination light to the light guide 131 of the electronic endoscope 100 to illuminate the living tissue T near the insertion tube tip portion 111 of the electronic endoscope 100.

In this embodiment, the light source unit 400 of the processor 200 for the electronic endoscope includes the light source 430, a collimator lens 440, a spectral filter 410, a filter control unit 420 and a condenser lens 450. The white light emitted from the light source 430 is converted by the collimator lens 440 into a collimated beam, passes through the spectral filter 410, and then is incident on the proximal end portion 131b of the light guide 131 by the condenser lens 450. The spectral filter 410 is a filter of a circular plate type which breaks down the white light from the light source 430 into a light of a predetermined wavelength (i.e., selects a wavelength), and selects and outputs lights of narrow bandwidths with wavelength of 400nm, 405nm, 410nm, ... , 800nm (bandwidths of approximately 5nm) depending on the rotation angle thereof. The rotation angle of the spectral filter 410 is controlled by the filter control unit 420 connected to the controller 530. Since the controller 530 controls the rotation angle of the spectral filter 410 via the filter control unit 420, lights with predetermined wavelengths are incident on the proximal end portion 131b of the light guide 131, and the living tissue T near the insertion tube tip portion 111 is illuminated. Then, lights reflected from the living tissue T are converged onto the light-receiving surface of the image pick-up device 141 as described above, and the image signals are transmitted to the image processing unit 500 via the cable 142.

The image processing unit 500 is a device which obtains a plurality of spectral images, at intervals of a wavelength of 5nm, from images of the living tissue T obtained via the cable 142. Specifically, when the spectral filter 410 selects and outputs the narrow bandwidth lights (a bandwidth of approximately 5nm) with the center wavelengths of 400nm, 405nm, 410nm,···, 800nm, spectral images with respective wavelengths are obtained.

The image processing unit 500 has a function of processing a plurality of spectral images obtained by the spectral filter 410 to generate color images or extracted lesion images as described later. And then the image processing unit 500 controls the image display device 300 to display the processed spectral images and the extracted lesion images.

Here, as the spectral filter 410, spectral filters (such as the Fabry-Perot filter) or well-known spectral image capturing methods which use transmission type diffraction gratings can be adopted to obtain spectrally dispersed light.

As described above, the image processing unit 500 in the embodiment has the function of generating the extracted lesion images by extracting the area with high probability of being lesions using a plurality of spectral images with different wavelengths. In the following, a function of generating the extracted lesion images is explained.

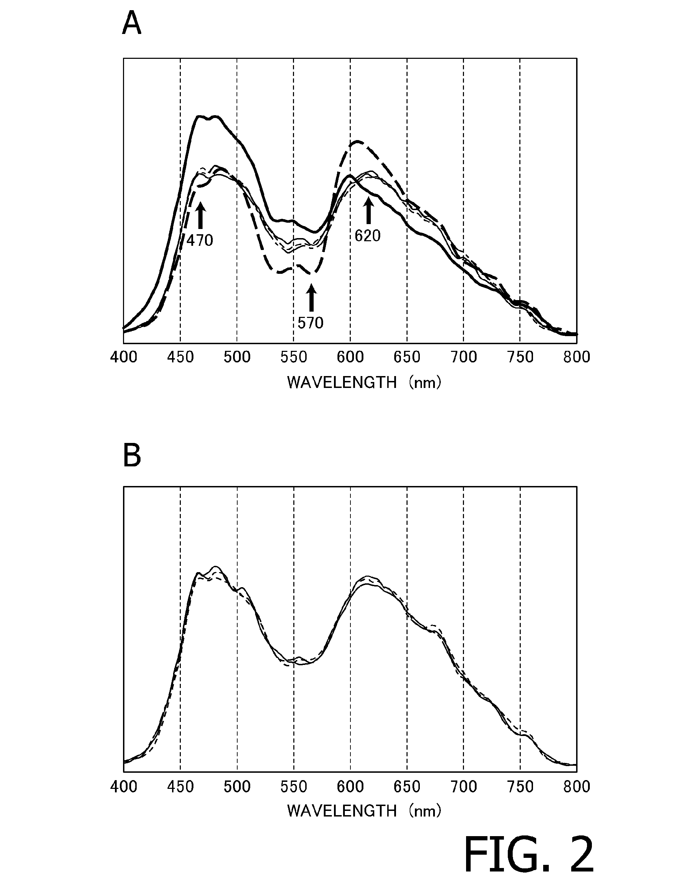

First, the principle of extracting areas with high probabilities of being lesions, and index values which are the basis of the extracted lesion images generated by the image processing unit 500 in the embodiment of the invention, are explained. Fig. 2 represents spectral image data of the gastric mucosa obtained by the diagnostic system 1 in the embodiment of the invention, and each waveform represents a spectrum of a particular pixel in the spectral images (i.e., brightness values for each wavelength). Fig. 2A represents a spectrum of a pixel corresponding to a lesion of the gastric mucosa, and Fig. 2B represents a spectrum of a pixel corresponding to a healthy region of the gastric mucosa. Here, for convenience of explanation, standardization process is applied to spectrum of each pixel of the healthy region and the lesion shown in Fig. 2. Specifically, since each pixel of the image pickup device 141 receives different amount of light due to angle differences between the illumination light and the object (living tissue T) and distance differences between the insertion tube tip portion 111 (Fig. 1) and the living tissue T, influences of these light amount differences are corrected.

As shown in Fig. 2, the spectrum of the gastric mucosa has, regardless of whether it is the healthy region or the lesion, a substantially M-shaped property with a valley extending in wavelengths of 500nm to 590nm. However, variability of the spectrum of pixels for the lesion is greater than that of the healthy region, and the spectrum of pixels for the lesion differs from that of the healthy region in that it has two valleys at wavelengths of about 540 nm and 570 nm. Therefore, it is possible to identify healthy regions and lesions by analyzing the spectrum of each pixel of the spectral images. However, since healthy regions and lesions normally lie next to each other, it is difficult to clearly identify boundaries between healthy regions and lesions by the shapes of the spectrums. For this reason, as explained below, the inventors of the invention found a configuration to identify healthy regions and lesions quantitatively using multiple regression coefficients derived from multiple regression analysis on the spectral image data.

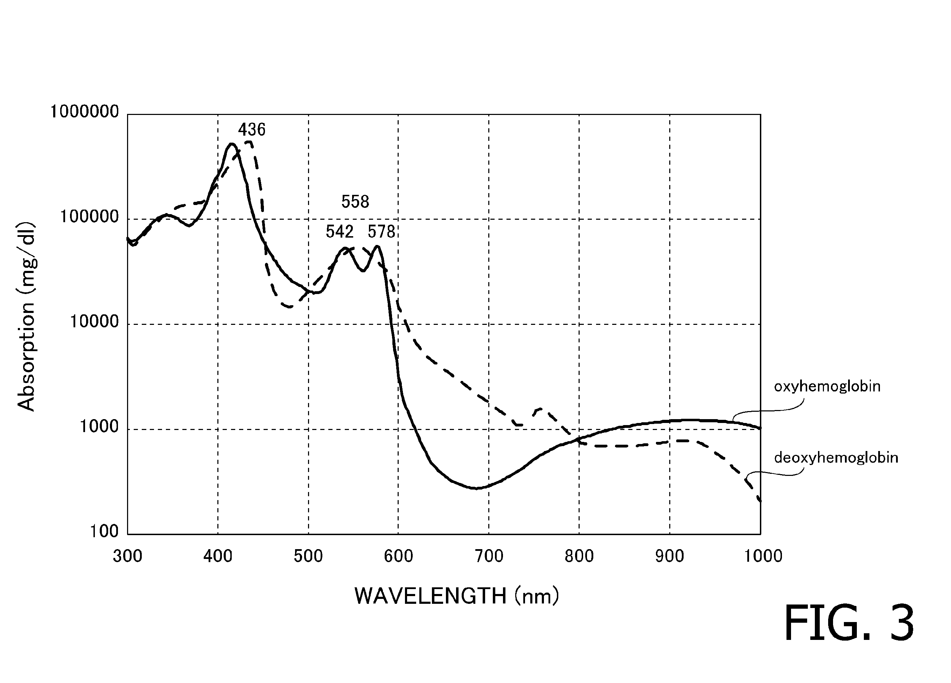

Fig. 3 is a graph representing light absorption properties of hemoglobin. A solid line represents a light absorption property of oxyhemoglobin, and a dashed line represents a light absorption property of deoxyhemoglobin. As shown in Fig. 3, oxyhemoglobin and deoxyhemoglobin are common in that they absorb lights with wavelengths of between 500nm to 590 nm (i.e., absorption properties increase at wavelengths of between 500nm to 590 nm), but differ in that deoxyhemoglobin has one peak at the wavelength of about 560 nm, whereas oxyhemoglobin has two peaks at the wavelengths of about 540 nm and 570 nm. The inventors of the invention focused on this property difference, and performed multiple regression analysis using the spectral image data of the gastric mucosa shown in Fig. 2 as dependent variables and the light absorption properties of oxyhemoglobin and deoxyhemoglobin as independent variables. As a result, the inventors of the invention found that the spectral image data of the gastric mucosa can be explained using the light absorption properties of oxyhemoglobin and deoxyhemoglobin, and that if the concentration of oxyhemoglobin at lesions is larger than that at healthy regions, quantitative identification of healthy regions and lesions based on multiple regression coefficient of oxyhemoglobin is possible. Furthermore, the embodiment of the invention is configured so that, in addition to absolute evaluation of spectral properties at one point (pixel), relative evaluation with the surrounding area can be performed, by using the two-dimensional spectral information. This configuration enables high detection accuracies of the lesions even when absolute evaluation is difficult due to influences of tissues, configurations, individual differences, and states of lesions in a living body.

In general, a measurement model of spectral image data obtained from the embodiment of the invention is expressed using Beer-Lambert Law as the following expression (1).

(EXPRESSION 1)

$$A(\lambda) = -\log_{10} \frac{I(\lambda)}{I_0(\lambda)} = \varepsilon(\lambda)Cd$$

In the expression (1), $A$ is an absorption coefficient of a medium (living tissue T), $I_0$ is a radiation intensity of light before entering a medium, $I$ is an intensity of light travelled in the medium for a distance of $d$, $\varepsilon$ is a molar light absorption coefficient, $C$ is a mol concentration, and $\lambda$ is a wavelength of light. If a medium has $n$ types of light-absorbing substances, then the absorption coefficient is expressed as the following expression (2).

(EXPRESSION 2)

$$A(\lambda) = \sum_{1}^{n} \varepsilon_i(\lambda) C_i d$$

That is, in case where the medium has $n$ types of light-absorbing substances, the absorption coefficient $A$ is expressed as a summation of absorption properties for each substance. As shown in an expression (3) below, multiple regression analysis is performed using the spectral data of the gastric mucosa shown in Fig. 2 as dependent variables and the light absorption properties of oxyhemoglobin and deoxyhemoglobin as independent variables.

(EXPRESSION 3)

$$\begin{bmatrix} X_{400} \\ X_{405} \\ \vdots \\ X_{800} \end{bmatrix} \equiv P1 \times \begin{bmatrix} a_{400} \\ a_{405} \\ \vdots \\ a_{800} \end{bmatrix} + P2 \times \begin{bmatrix} b_{400} \\ b_{405} \\ \vdots \\ b_{800} \end{bmatrix}$$

In expression (3), $X$ are data for one pixel of spectral images of the gastric mucosa, and represent brightness values of the spectral images captured by irradiating lights with central wavelengths of 400nm to 800nm at intervals of a wavelength of 5nm. The values $a$ are the light absorption properties of oxyhemoglobin for wavelengths of 400 nm to 800 nm captured at every 5 nm, and the values $b$ are the light absorption properties of deoxyhemoglobin for wavelengths of 400nm to 800nm captured at intervals of a wavelength of 5nm. Then, multiple regression analysis is performed by resolving the expression (3) for the multiple regression coefficients P1 and P2.

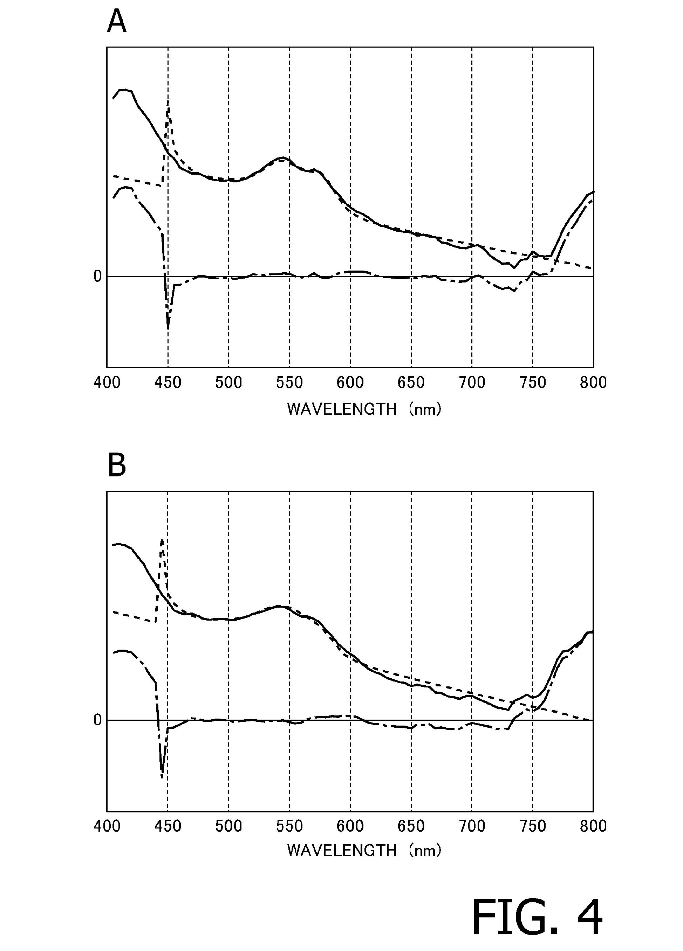

Fig. 4 is a graph representing results of multiple regression analysis on the spectral image data of the gastric mucosa shown in Fig. 2. Fig. 4A is a graph showing a result of multiple regression analysis on the spectrum of a pixel corresponding to the lesion of the gastric mucosa shown in Fig. 2A after the vertical axis being converted to absorption property, and Fig. 4B is a graph showing a result of multiple regression analysis on the spectrum of a pixel corresponding to the healthy region of the gastric mucosa shown in Fig. 2B after the vertical axis being converted to absorption property. In Fig. 4A and Fig. 4B, solid lines represent data series for the spectral image data of the gastric mucosa, dashed lines represent data series for the result of the multiple regression analysis, and chain lines represent data series for residuals after the multiple regression analysis (i.e., differences between the results of the multiple regression analysis and the spectral image data). As shown in Fig. 4, regardless of whether it is the healthy region or the lesion, each waveform in Fig. 2 (i.e., a spectrum of a specific pixel in the spectral image) can be substantially expressed by a combination of the light absorption properties of oxyhemoglobin and deoxyhemoglobin. Here, as a measured model of spectral image data obtained in the embodiment, scattered lights when lights are incident on the living tissue T are called into account, but the addition of the scattered lights are omitted in the expression 3. By carrying out the multiple regression analysis described above, it turned out that a spectrum of a predetermined pixel in the spectral image can be explained using a combination of the light absorption properties of oxyhemoglobin and deoxyhemoglobin with substantially no residual errors.

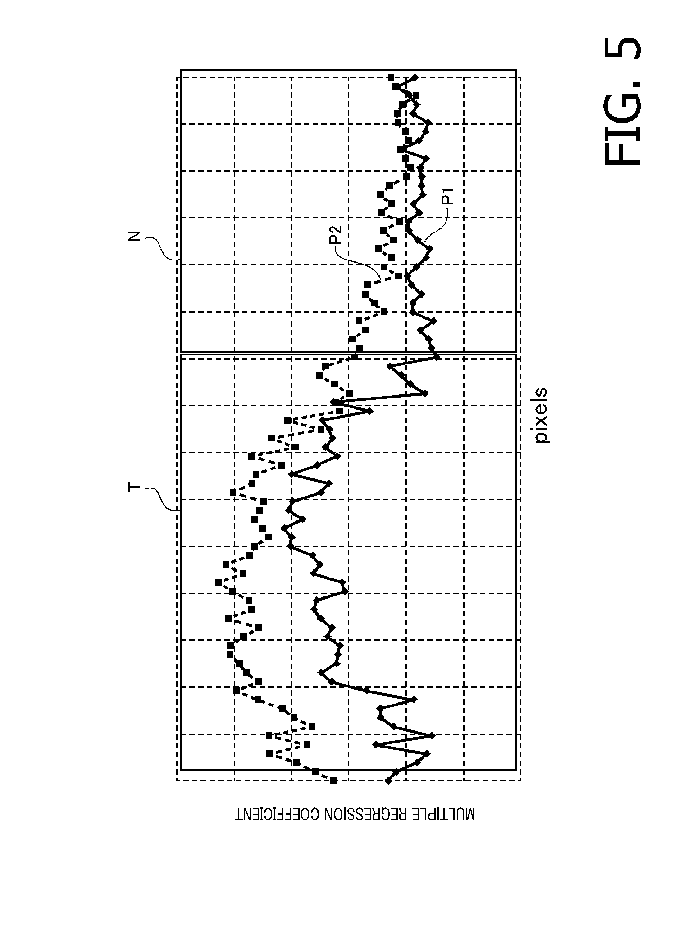

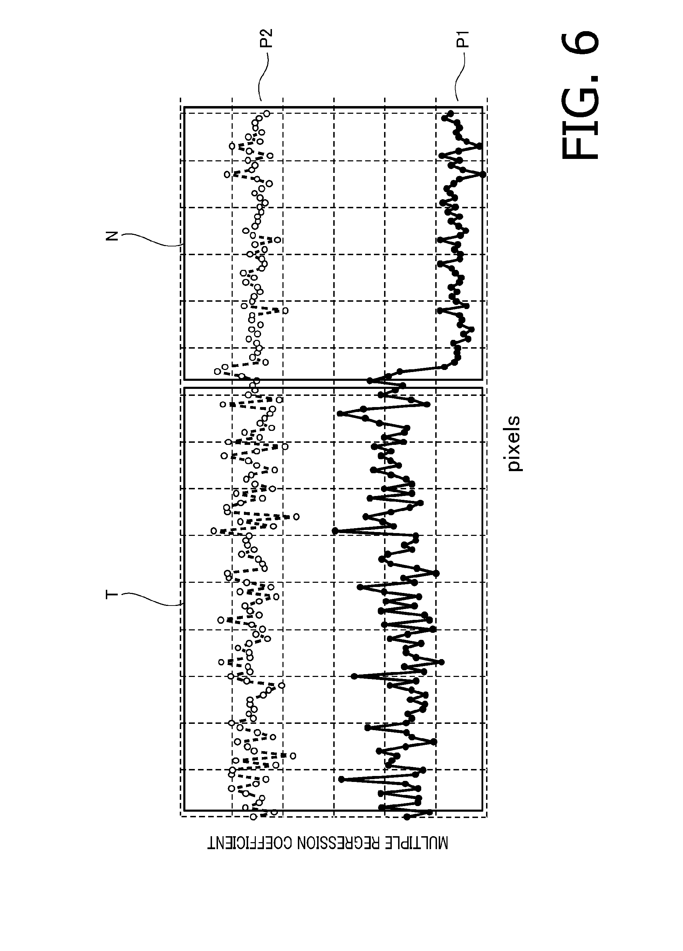

Fig. 5 is a graph representing the first example of multiple regression coefficients P1 and P2 obtained from multiple regression analysis on the spectral image data of the gastric mucosa shown in Fig. 2. Fig. 6 is a graph representing the second example of multiple regression coefficients P1 and P2 obtained from multiple regression analysis on the spectral image data of the gastric mucosa shown in Fig. 2. A range indicated by frame T in Fig. 5 and Fig. 6 indicates multiple regression coefficients P1 and P2 corresponding to a pixel of the lesion, and a range indicated by frame N indicates multiple regression coefficients P1 and P2 corresponding to a pixel of the healthy region. In the example shown in Fig. 5, it is observed that dispersion of the multiple regression coefficients P1 and P2 corresponding to pixels of the lesion is larger than that of the healthy region, and the multiple regression coefficients P1 and P2 corresponding to pixels of the lesion are larger than that corresponding to the healthy region. Here, from the expression 3, the multiple regression coefficient P1 is a parameter representing the amount of oxyhemoglobin (i.e., concentrations), and the multiple regression coefficient P2 is a parameter representing the amount of deoxyhemoglobin. Therefore, the result indicates that larger amount of oxyhemoglobin and deoxyhemoglobin is detected from the lesion than from the healthy region in the example shown in Fig. 5. Furthermore, in the example shown in Fig. 6, it is observed that dispersion of the multiple regression coefficients P1 and P2 for pixels of the lesion is larger than that of the healthy region, and the multiple regression coefficient P1 for the pixels of the lesion is larger than that of the healthy region. As explained above, from the experiment by the inventors of the invention, two trends shown in Fig. 5 and Fig. 6 are observed. Furthermore, from the previous studies, it is known that in lesions such as cancer, a sum of amount of oxyhemoglobin and deoxyhemoglobin (corresponding to a total detected amount of blood) and a ratio of deoxyhemoglobin against oxyhemoglobin are greater than those in healthy regions. Therefore, as shown in Fig. 5, examples showing that a sum of the multiple regression coefficients P1 and P2 and a ratio of the multiple regression coefficient P2 against the multiple regression coefficient P1 become larger in lesions, are dominant in general. Thus, in this embodiment, ratio R of multiple regression coefficients P1 and P2 is derived using the expression (4) below, and the ratio is used as an index value to identify lesions and healthy regions. The image processing unit 500 according to the embodiment generates the extracted lesion images from this index value.

(EXPRESSION 4)

$$R = P1/P2$$

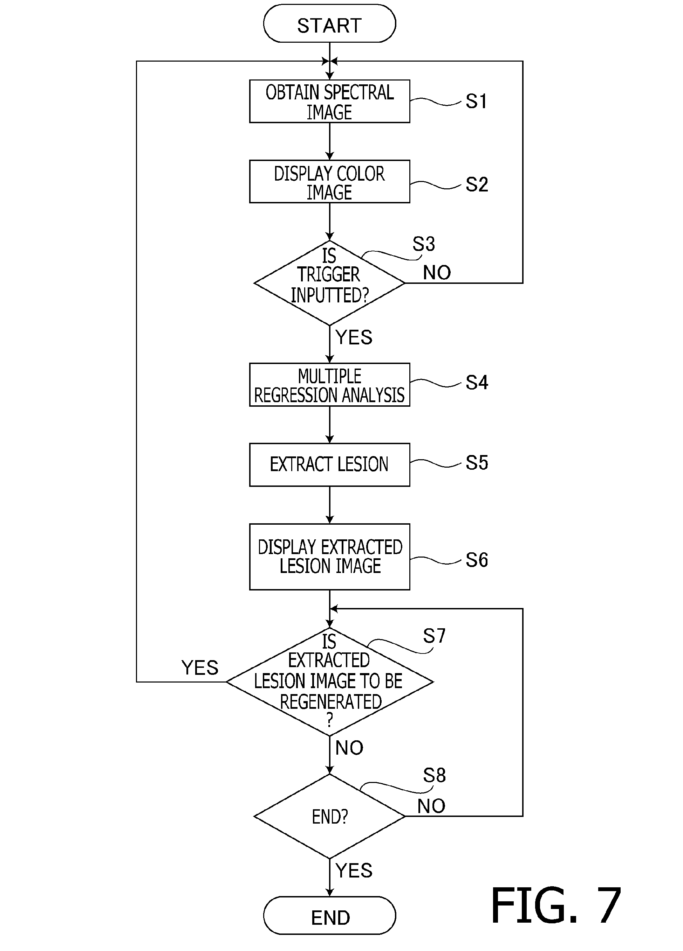

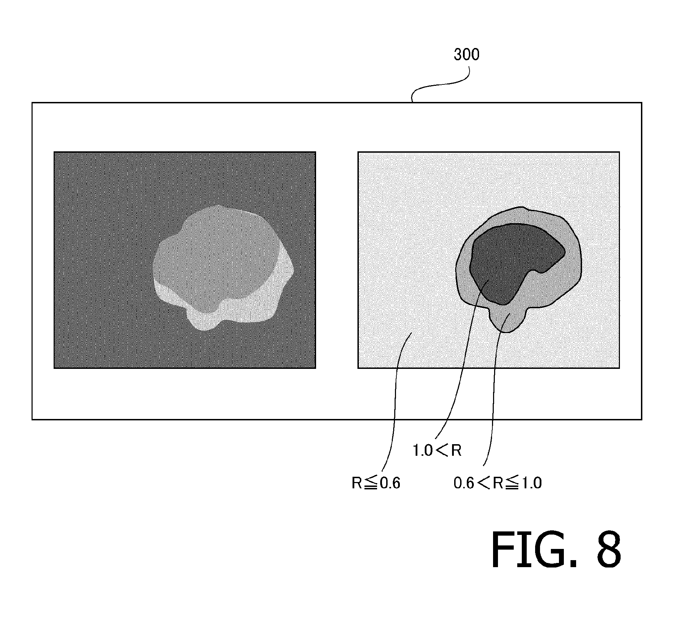

In the following, an image generating process executed by the image processing unit 500 in the embodiment is explained. Fig. 7 is a flowchart of the image generating process executed by the image processing unit 500 of the embodiment, and Fig. 8 illustrates a color image and an extracted image of a lesion generated by the image generating process shown in Fig. 7 and displayed on the image display device 300. The image generating process is a routine to generate the color images and the extracted lesion images and to display on the image display device 300. This routine is executed at a time of power-on of the diagnostic system 1.

When the routine is started, step S1 is executed. In step S1, the image processing unit 500 transmits a control signal for controlling the filter control unit 420 to obtain the spectral image. When receiving the control signal, the filter control unit 420 controls the rotation angle of the spectral filter 410 so as to sequentially select lights of narrow bands (a bandwidth of approximately 5nm) with wavelengths of 400nm, 405nm, 410nm, ··· 800nm. The image processing unit 500 captures the spectral image obtained at each wavelength and stores the spectral image in the temporary memory 520. Then, the process proceeds to step S2.

In step S2, three images having the center wavelengths of 435nm, 545nm and 700nm are extracted from the spectral images obtained at step S1, and one piece of color image data in which an image of the center wavelength of 435nm is stored in a blue plane, an image of the center wavelength of 545nm is stored in a green plane and an image of the center wavelength of 700nm is stored in a red plane, is generated. As described above, the color image data is obtained from a spectral image of a blue color wavelength of 435nm, a spectral image of a green color wavelength of 545nm and a spectral image of a red color wavelength of 700nm, and so is a color image equivalent to the endoscopic image from normal observation. Then, the image processing unit 500 transmits the generated color image data to the video memory 540, and displays the color image on the left side of the screen of the image display device 300 (Fig. 8). Then, the process proceeds to step S3.

In step S3, it is checked whether a trigger input designating a generation of the extracted lesion images occurs through use of an operating unit (not shown) of the processor 200 for the electronic endoscope during execution of steps S1 and S2. When the trigger input does not occur (S3: NO), the process proceeds to step S1 to obtain the spectral image again. That is, unless the trigger input occurs, the color image obtained from the spectral image is sequentially updated and is continuously displayed on the image display device 300. On the other hand, when the trigger input occurs during the execution of steps S1 and S2 (S3: YES), the process proceeds to step S4.

In step S4, multiple regression analysis on the spectral image obtained in step S1 is executed. Specifically, the multiple regression coefficients P1 and P2 for all the pixels in the spectral image obtained in step S1 are calculated using the expression (3). Then, the process proceeds to step S5.

In step S5, the index values (ratio R) of the multiple regression coefficients P1 and P2 for each pixel derived in step S4 are calculated using the expression (4). Then, the process proceeds to step S6.

In step S6, the extracted lesion image is generated using the index value for each pixel obtained in step S5. Specifically, a predetermined color is assigned to each pixel according to the index value for each pixel to form the extracted lesion image. In this embodiment, pixels with the index values (the ratio R) equal to or lower than 0.6 are judged healthy regions and blue color is assigned, pixels with the index values greater than 0.6 and equal to or lower than 1.0 are judged as the boundaries between the healthy regions and the lesions and green color is assigned, and the pixels with the index values greater than 1.0 are judged lesions and red color is assigned. The extracted lesion image thus generated is displayed on the right side of the screen of the image display device 300 (Fig. 8). By displaying the extracted lesion image, which is color-coded according to the index values, and the color image of the endoscopic image next to each other on the screen of the image display device 300, the user of the diagnostic system 1 can identify which regions in the color image are the lesions by comparing the extracted lesion image with the color image. Then, the process proceeds to step S7.

In step S7, the image processing unit 500 displays on the image display device 300 a message inquiring whether to regenerate the extracted lesion image, and in the mean time accepts the input from the operating unit (not shown) of the processor 200 for the electronic endoscope. When the user of the diagnostic system 1 operates the operating unit selecting a regeneration of the extracted lesion image (S7: YES), the process returns to step S1. On the other hand, when an input for regenerating the extracted lesion image is not made for a predetermined period of time (e.g., for several seconds) (S7: NO), the process proceeds to step S8.

In step S8, the image processing unit 500 displays on the image display device 300 the message inquiring whether to terminate displaying the extracted lesion image, and in the mean time accepts the input from the operating unit (not shown) of the processor 200 for the electronic endoscope. When the user of the diagnostic system 1 operates on the operating unit selecting the termination of displaying of the extracted lesion image (S8: YES), the routine is terminated. On the other hand, when an input for displaying the extracted lesion image is not made for a predetermined period of time (e.g., for several seconds) (S8: NO), the process proceeds to step S7.

As described above, by executing the routine shown by the flowchart in Fig. 7 through the image processing unit 500, the extracted lesion images which are useful to estimate the position of the lesions are displayed on the image display device 300. By displaying the regions with high probabilities of being lesions as the extracted lesion images in the aforementioned manner, doctors can diagnose by identifying the position or area of lesions, and by comparing with the tissue around them.

As described above, in this embodiment, the index value (the ratio R) for each pixel is calculated from the multiple regression coefficients P1 and P2 using the expression (4), and the area (pixels) with high probabilities of being lesions are determined by the index values. However, the present invention is not limited to the above described configuration. For example, the area (pixels) with high probabilities of being lesions can be determined using the amplitudes of the multiple regression coefficients P1 as index values.

Furthermore, in this embodiment, the image processing unit 500 is configured to perform multiple regression analysis using the spectral image data obtained in the wavelength range of 400nm to 800nm at intervals of wavelength of 5nm, but the present invention is not limited to this configuration. For example, the wavelength range can be set narrower, including the wavelength band of 500nm to 590nm which is the absorption wavelength band of the oxyhemoglobin and deoxyhemoglobin, and the standard wavelengths needed to standardize each pixel. It can also be configured to perform the multiple regression analysis using only the spectral images obtained from the wavelengths of 500 nm to 590 nm which is the absorption wavelength band of the oxyhemoglobin and deoxyhemoglobin. It does not need to be configured to obtain the spectral image data at intervals of wavelength of 5nm, as long as the spectrum of pixels corresponding to the lesions and that corresponding to the healthy regions can be identified. For example, the interval of wavelength for obtaining the spectral image data can be selectable within the range of 1 to 10 nm.

In this embodiment, it is configured so that the image processing unit 500 assigns predetermined colors to each pixel of the spectral images to obtain the extracted lesion images, but this invention is not limited to this configuration. For example, it can be configured to compare the index values with a predetermined threshold value, determining that the probabilities of being lesions are high if the index values are greater than the threshold value (i.e., large amount of oxyhemoglobin is detected), and extract the corresponding pixels to form the extracted lesion images. More specifically, compare the index value of each pixel with the predetermined threshold value, assign "1" to the pixel if the index value is greater than the predetermined threshold value, or assign "0" to the pixel if the index value is smaller than the predetermined threshold value, to form a two dimensional binary image.

**Claims**

1. A diagnostic system, comprising:

   a spectral imaging means that captures a spectral image within a predetermined wavelength range in a body cavity and obtains spectral image data;
   an image processing means that receives the spectral image data, determines an index value indicating a region that is highly likely to be a lesion from the spectral image data, and generates and outputs an extracted lesion image based on the index value; and
   a monitor on which the extracted lesion image is displayed,
   wherein the image processing means performs multiple regression analysis for each pixel of the spectral image with the spectral image data as a dependent variable and respective light absorption properties of oxyhemoglobin and deoxyhemoglobin as independent variables, and determines the index value based on respective concentrations of the oxyhemoglobin and the deoxyhemoglobin.

2. The diagnostic system according to claim 1, wherein the image processing means determines a ratio between the concentration of the oxyhemoglobin and the concentration of the deoxyhemoglobin as the index value.

3. The diagnostic system according to claim 1 or 2, wherein the image processing means generates the extracted lesion image by assigning to each pixel of the spectral image a predetermined color according to the index value.

4. The diagnostic system according to claim 1 or 2,
   wherein the image processing means comprises:

   a comparing means that compares the index value with a predetermined threshold value; and
   a binary image generating means that generates a binary image based on a result of the comparison by the comparing means, and
wherein the extracted lesion image is generated based on the binary image.

5. The diagnostic system according to any of claims 1 to 4,
    wherein the image processing means generates a color image by synthesizing data, of the spectral image data, having wavelength bands for blue color, green color and red color, and outputs the color image, and
    wherein, on the monitor, the color image and the extracted lesion image are displayed next to each other.

6. The diagnostic system according to any of claims 1 to 5, wherein the image processing means determines the index value from data, of the spectral image data, having a wavelength band of 500 nm to 590 nm that is an absorption wavelength band of the oxyhemoglobin and the deoxyhemoglobin.

7. The diagnostic system according to any of claims 1 to 6,
    wherein the predetermined wavelength range is from 400 nm to 800 nm, and
    the spectral image includes a plurality of images captured at every predetermined wavelength range of 1 nm to 10 nm.

FIG. 1

A

470

570

620

WAVELENGTH (nm)

B

WAVELENGTH (nm)

FIG. 2

FIG. 3

FIG. 4

FIG. 5

FIG. 6

START

OBTAIN SPECTRAL IMAGE — S1

DISPLAY COLOR IMAGE — S2

IS TRIGGER INPUTTED? — S3 — NO

YES

MULTIPLE REGRESSION ANALYSIS — S4

EXTRACT LESION — S5

DISPLAY EXTRACTED LESION IMAGE — S6

IS EXTRACTED LESION IMAGE TO BE REGENERATED? — S7 — YES

NO

END? — S8 — NO

YES

END

FIG. 7

300

1.0＜R

R≦0.6          0.6＜R≦1.0

FIG. 8

## INTERNATIONAL SEARCH REPORT

| | International application No. |
|---|---|
| | PCT/JP2012/053093 |

A. CLASSIFICATION OF SUBJECT MATTER
*A61B1/00*(2006.01)i, *A61B1/04*(2006.01)i, *A61B5/1455*(2006.01)n

According to International Patent Classification (IPC) or to both national classification and IPC

B. FIELDS SEARCHED

Minimum documentation searched (classification system followed by classification symbols)
A61B1/00, A61B1/04, A61B5/1455

Documentation searched other than minimum documentation to the extent that such documents are included in the fields searched
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2003-508735 A (Nederlandse Organisatie voor Toegepast-natuurwetenschappelijk Onderzoek TNO), 04 March 2003 (04.03.2003), entire text; all drawings & US 6775565 B1 & EP 1207781 A & WO 2001/015597 A1 & WO 2001/015597 B & DE 60024836 D & DE 60024836 T & NL 1012943 C & AU 7323600 A & AT 312548 T & ES 2254217 T & NL 1012943 C2 | 1-7 |
| Y | JP 2009-068940 A (Canon Inc.), 02 April 2009 (02.04.2009), entire text; all drawings & US 2009/0069653 A1 & EP 2036489 A2 | 1-7 |

| ☒ | Further documents are listed in the continuation of Box C. | ☐ | See patent family annex. |

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 29 February, 2012 (29.02.12) | 13 March, 2012 (13.03.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2012/053093

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 2009-66301 A  (Fujifilm Corp.),<br>02 April 2009 (02.04.2009),<br>entire text; all drawings<br>(Family: none) | 4-6 |
| A | JP 2009-131617 A  (Fujifilm Corp.),<br>18 June 2009 (18.06.2009),<br>paragraphs [0078], [0079]<br>& US 2009/0122152 A1    & EP 2057935 A1 | 1-7 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004321792 A